# EUROPÄISCHE PATENTANMELDUNG

(11) **EP 1 253 556 A2**
(43) Veröffentlichungstag der Anmeldung: **30.10.2002**
(21) Anmeldenummer: 02100194.6
(22) Anmeldetag: 27.02.2002
(51) Int. Cl.: G06T 5/00

(54) **Verfahren und Vorrichtung zur Bildverarbeitung von Röntgenaufnahmen**

(30) Priorität: 28.02.2001 DE 10109586
(71) Anmelder: Philips Corporate Intellectual Property GmbH, 20099 Hamburg (DE); Koninklijke Philips Electronics N.V., 5621 BA Eindhoven (NL)
(72) Erfinder: Schmitz, Georg, 52066, Aachen (DE); Braess, Henning, 52066, Aachen (DE); Reiter, Harald, 52066, Aachen (DE)
(74) Vertreter: Volmer, Georg, Dipl.-Ing.

(57) **Zusammenfassung**

Die Erfindung betrifft ein Verfahren und eine Vorrichtung zur Bildverarbeitung von Röntgenaufnahmen, welche insbesondere bei medizinischen Durchleuchtungsprozeduren eingesetzt werden können, da sie die gesamte Strahlenbelastung für einen Patienten gering halten. Zur Bildverarbeitung und Detailverstärkung der Röntgenaufnahmen wird vorzugsweise ein Pattern-Matching-Algorithmus eingesetzt, welcher die Kenntnis eines Musters des interessierenden Details voraussetzt. Zur Gewinnung dieses Musters wird mindestens eine Einzelaufnahme (HD) höherer Dosisleistung angefertigt (2), welche eine ausreichende Bildqualität für die automatische (4) oder nicht-automatische (3) Erkennung des Musters aufweist. Die aus dieser Einzelaufnahme gewonnenen Informationen dienen dann als Basis für die Auswertung von Röntgenaufnahmen (LD2) geringerer Dosisleistung.

## Beschreibung

Die Erfindung betrifft ein Verfahren zur Bildveraibeitung von Röntgenaufnahmen und eine Vorrichtung zur Durchführung dieses Verfahrens.

Mit Hilfe von Röntgenstrahlung hergestellte Durchleuchtungsbilder von Objekten erreichen in der Regel ein um so besseres Signal-Rausch Verhältnis und damit eine um so bessere Bildqualität, je höher die Dosisleistung der verwendeten Röntgenstrahlung ist. Einer aus diesen Gründen an sich wünschenswerten Verwendung hoher Dosisleistungen bei der Aufnahme von Röntgenbildern steht jedoch die steigende Strahlenbelastung des untersuchten Objektes entgegen. Dies gilt in besonderem Maße bei der Herstellung von medizinischen Röntgenaufnahmen an einem Patienten, bei denen aus gesundheitlichen Gründen die Strahlenbelastung so gering wie möglich zu halten ist. Vor allem bei Durchleuchtungsuntersuchungen (Fluoroskopie), bei welchen mit einer Wiederholungsfrequenz von typischerweise 30 Bildern pro Sekunde Aufnahmen von einem Patienten gemacht und auf einem Bildschirm dargestellt werden, ist die Begrenzung der eingesetzten Dosisleistung auf vertretbare Werte zu beachten. Um dennoch aus den gewonnenen Röntgenaufnahmen ein Maximum an Information herauszuholen, werden daher möglichst leistungsstarke Algorithmen zur Bildverarbeitung eingesetzt, welche Störeffekte unterdrücken und die interessierenden Details verbessert darstellen sollen.

So beschreibt z.B. die US 5 289 373 ein Verfahren zur Erkennung und Hervorhebung eines Führungsdrahtes (guide wire) auf einem Durchleuchtungsbildschirm, wobei der Führungsdraht von einem in das Gefäßsystem eines Patienten eingeführten Katheter ausgeht. Mit dem Algorithmus wird ein Bildbereich, in dem sich der Führungsdraht befinden soll, automatisch untersucht, wobei zunächst die Pixel bestimmt werden, an denen lokale Extrema der Grauwerte vorliegen, und wobei dann ermittelt wird, welche dieser Pixel eine zusammenhängende Linie ergeben, die dem Führungsdraht entsprechen könnte.

Der aus der US 5 289 373 bekannte Algorithmus benötigt über das darzustellende Detail "Führungsdraht" keine weiteren Informationen als die allgemeinen Eigenschaften, dass sich der Führungsdraht durch einen Schatten (Grauwertextremum) darstellt und eine zusammenhängende Linie ergeben muss. Da auch andere Objekte als der gesuchte Führungsdraht diese Kriterien erfüllen können, unterliegt der Algorithmus jedoch der Gefahr, die falsche Struktur als den Führungsdraht zu identifizieren. Weiterhin kann es sein, dass der Algorithmus den Führungsdraht nicht finden kann, da dieser in der speziellen Bildaufnahme die vorausgesetzten Kriterien ausnahmsweise nicht erfüllt. Zur Erhöhung der Wahrscheinlichkeit, dass der Algorithmus den Führungsdraht korrekt detektiert, ist es daher vorteilhaft, wenn das zu durchsuchende Gebiet durch den Anwender möglichst eng eingeschränkt werden kann.

Weiterhin sind zur Erkennung von bestimmten Objekten beziehungsweise Strukturen leistungsfähige Algorithmen entwickelt worden, welche ein vorgegebenes Muster der zu erkennenden Struktur verwenden. Die Algorithmen suchen dann in einer Röntgenaufnahme diejenigen Stellen, welche am besten zu dem vorgegebenen Muster ("pattern") passen. Aufgrund dieser Arbeitsweise werden solche Algorithmen auch als "Pattern-Matching-Algorithmen" bezeichnet. Nachteilig bei solchen Algorithmen ist allerdings, dass sie ein Muster des gesuchten Details benötigen, welches gerade bei dynamischen Objekten wie einem durch den Körper bewegten Führungsdraht nicht immer bzw. nicht gleichbleibend verfügbar ist.

Vor diesem Hintergrund war es Aufgabe der vorliegenden Erfindung, ein Verfahren und eine Vorrichtung zur Bildverarbeitung von Röntgenaufnahmen bereitzustellen, welche bei Minimierung der eingesetzten Dosisleistung der Röntgenstrahlung eine gute Erkennungsleistung und Bildverbesserung für interessierende Details ergeben.

Diese Aufgabe wird durch ein Verfahren mit den Merkmalen des Anspruchs 1 und eine Vorrichtung mit den Merkmalen des Anspruchs 7 gelöst. Vorteilhafte Ausgestaltungen sind in den Unteransprüchen enthalten.

Bei dem Verfahren zur Bildverarbeitung von Röntgenaufnahmen - insbesondere von medizinischen Röntgenbildern aus einer Durchleuchtung - wird mindestens eine Röntgenaufnahme mit einer Dosisleistung hergestellt, die im Vergleich zu den bei den übrigen Aufnahmen verwendeten Dosisleistungen höher ist. Die Aufnahme besitzt daher ein besseres Signal-Rausch Verhältnis beziehungsweise eine höhere Bildqualität. Diese Einzelaufnahme höherer Bildqualität wird dann als Basis für den Algorithmus zur Bildverarbeitung der übrigen (früheren und/oder insbesondere nachfolgenden) Aufnahmen verwendet.

Das erfindungsgemäße Verfahren kann insgesamt mit einer geringen Strahlenbelastung des untersuchten Objektes wie insbesondere eines durchleuchteten Patienten arbeiten, da die überwiegende Mehrzahl der Röntgenaufnahmen bei geringer Dosisleistung hergestellt wird. Nur einmal beziehungsweise nur verhältnismäßig selten werden Einzelaufnahmen mit höherer Dosisleistung erstellt. Typischerweise kommen auf eine solche Einzelaufnahme mehrere Hundert oder sogar Tausend gewöhnliche Röntgenaufnahmen geringerer Dosisleistung. Die erhöhte Dosisleistung der Einzelaufnahmen fällt daher insgesamt für die Strahlenbelastung kaum ins Gewicht. Für die Bildverarbeitung aller Röntgenaufnahmen sind die Einzelaufnahmen aufgrund ihrer höheren Qualität jedoch ein bedeutender Fortschritt. Insbesondere kann aus diesen Einzelaufnahmen eine Information gewonnen werden, die in den normalen Aufnahmen nicht vorhanden ist und die dem eingesetzten Bildverarbeitungsalgorithmus dazu verhilft, die normalen Röntgenaufnahmen besser auswerten zu können.

Als Algorithmus zur Bildverarbeitung wird insbesondere ein Pattern-Matching-Algorithmus verwendet, welcher wenigstens ein Muster von dem darzustellenden beziehungsweise interessierenden Bilddetail wie etwa einem Führungsdraht verwendet. Dieses Muster kann dann aus den Einzelaufnahmen höherer Dosisleistung gewonnen werden, so dass auch in dynamischen Situationen stets ein verhältnismäßig aktuelles und präzises Muster zur Verfügung steht.

Gemäß einer bevorzugten Ausgestaltung des Verfahrens kann der Pattern-Matching-Algorithmus zusätzlich eine automatische Mustererkennungsprozedur einsetzen, um das für die Verarbeitung von Aufnahmen mit geringerer Dosisleistung benötigte Muster aus der Einzelaufnahme höherer Dosisleistung zu gewinnen. Das benötigte Muster muss dem Algorithmus daher nicht unter Einsatz von Vorwissen vorgegeben werden, sondern er kann es sich selbst aus den bereitgestellten Aufnahmen höherer Dosisleistung extrahieren. Dies ist insbesondere in dynamischen Situationen vorteilhaft, in denen sich das zu erkennende Muster in seiner Form ständig ändert. Ein Beispiel hierfür ist die Verfolgung eines Stents oder des Führungsdrahtes eines Katheters bei einer medizinischen Röntgendurchleuchtung.

Zusätzlich oder alternativ kann in dem Pattern-Matching-Algorithmus eine Eingabeschnittstelle vorgegeben sein, über welche das für die Verarbeitung von Aufnahmen mit geringerer Dosisleistung benötigte Muster vorgegeben werden kann, nachdem dieses extern aus einer Röntgenaufnahme höherer Dosisleistung extrahiert worden ist. Die externe Extraktion des vorzugebenden Musters kann dabei zum Beispiel durch den behandelnden Arzt geschehen. Auf diese Weise kann menschliches Wissen und menschliche Erkennungsleistung in den Pattern-Matching-Algorithmus einfließen und dann nachfolgenden Auswertungen von "normalen" Röntgenaufnahmen zugute kommen.

Weiterhin kann bei dem Verfahren vorgesehen sein, dass sich die Bildverarbeitung von Röntgenaufnahmen geringerer Dosisleistung auf solche Bereiche der Aufnahme konzentriert, in denen in der Röntgenaufnahme höherer Dosisleistung ein darzustellendes Detail detektiert wurde. Mit Hilfe der Einzelaufnahme höherer Qualität kann somit automatisch oder extern durch einen Anwender eine "Region of Interest" (ROI) festgelegt werden, auf die eine genauere Betrachtung konzentriert werden soll. Die zur Bildverarbeitung eingesetzten Algorithmen können dann aus verschiedenen Gründen eine bessere Leistung erzielen, wenn sie auf die wirklich interessierenden Bereiche beschränkt werden. Nicht zuletzt kann ein kleinerer Bereich schneller bearbeitet werden, was in Echtzeit arbeitenden Verfahren mehr Zeit für eine genauere Bildverarbeitung gibt, zB. für die Durchführung mehrerer Iterationen. Auch kann die Darstellung des interessierenden Bereiches verbessert werden, indem zum Beispiel das lokale Histogramm der Grauwerte auf das Darstellungsspektrum des eingesetzten Monitors gestreckt wird.

Der Zeitpunkt, zu dem eine Einzelaufnahme höherer Dosisleistung hergestellt wird, kann von einem Anwender extern vorgegeben werden. So kann beispielsweise ein Arzt eine solche Aufnahme auslösen, wenn er weiß, dass sich die Lage eines Katheters gegenüber der letzten Einzelaufnahme höherer Dosis erheblich verändert hat. Darüber hinaus ist es jedoch auch möglich, dass eine Röntgenaufnahme höherer Dosisleistung automatisch dann aufgenommen wird, wenn ein Performance-Kriterium, das ein Maß für die Güte beziehungsweise Zuverlässigkeit der eingesetzten Bildverarbeitung darstellt, eine vorgegebene Schwelle unterschreitet. Wenn zB. das Ergebnis des Pattern-Matching-Algorithmus zu schlecht wird, kann eine neue Einzelaufnahme höherer Dosisleistung angefertigt werden, um dem Pattern-Matching-Algorithmus wieder ein neues Muster als Ausgangsbasis zur Verfügung zu stellen.

Die Erfindung betrifft weiterhin eine Vorrichtung zur Bildverarbeitung von digitalisierten Röntgenaufnahmen, bei der es sich insbesondere um eine Vorrichtung zur Durchleuchtung eines Patienten für medizinische Zwecke handeln kann. Die Vorrichtung enthält einen Zentralprozessor, der so eingerichtet ist, dass er ein Verfahren der oben erläuterten Art ausrühren kann. Das heißt, dass der Zentralprozessor Zugriff auf die digitalisierten Röntgenaufnahmen hat und diese mit einem geeigneten Verfahren wie insbesondere einem Pattern-Matching-Algorithmus bearbeitet. Ferner ist der Zentralprozessor so eingerichtet, dass er Einzelaufnahmen, die bei einer höheren Dosisleistung als die übrigen Aufnahmen entstanden sind, als Ausgangsbasis für den eingesetzten Algorithmus verwenden kann. So kann zum Beispiel aus den Einzelaufnahmen höherer Dosisleistung das Muster automatisch detektiert werden, mit welchem ein Pattern-Matching-Algorithmus arbeitet, oder es kann die "Region of Interest" detektiert werden, auf welche sich die nachfolgende Bildverarbeitung des Zentralprozessors konzentrieren soll.

Weiterhin kann der Zentralprozessor mit der Steuerung eines Röntgenaufnahmegerätes derart verbunden sein, dass er dieser Steuerung eine Anforderung für eine Röntgenaufnahme höherer Dosisleistung übermitteln kann. Wenn z.B. die Ergebnisse der Bildverarbeitung eine vorgegebene Qualität nicht mehr erreichen, kann der Zentralprozessor ein Anforderungssignal an das Röntgenaufnahmegerät senden, woraufhin dieses eine Einzelaufnahme höherer Dosisleistung und damit besserer Qualität erstellt.
Im Folgenden wird die Erfindung mit Hilfe der Figur beispielhaft erläutert. Die einzige Figur zeigt schematisch den Ablauf eines erfindungsgemäßen Verfahrens zur Bildverarbeitung von Röntgenaufnahmen.

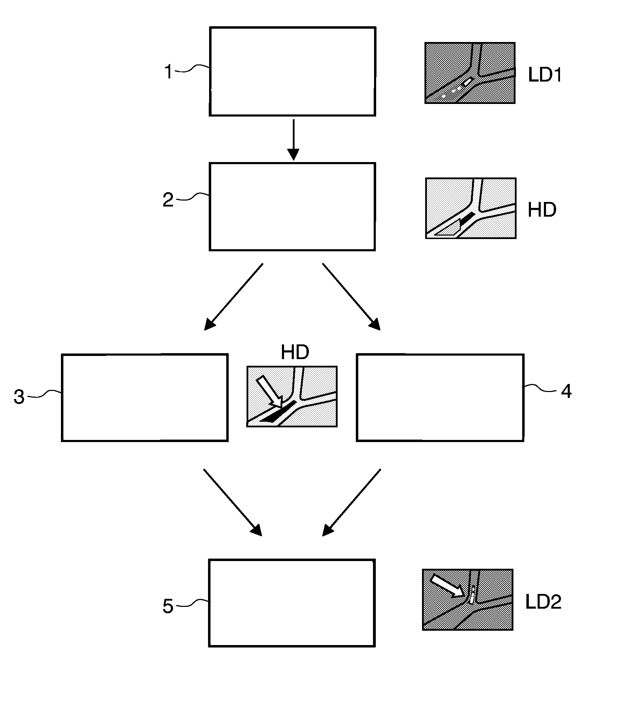

Das in Figur 1 dargestellte Verfahren ist vorteilhaft im Bereich der Bildverarbeitung von medizinischen Röntgendurchleuchtungen (Fluoroskopie) anwendbar. Hierbei geht es insbesondere um den Aspekt der Sichtbarkeit von kleinen Details wie zum Beispiel Führungsdrähten und Stents. Mit dem Verfahren lassen sich dabei die besonders leistungsstarken Algorithmen des Pattern-Matching anwenden, die voraussetzen, dass bekannt ist, wie das zu verbessernde Bilddetail aussieht. Beispiele für derartige Algorithmen sind beschrieben in I. Kompatsiaris et al.: "Deformable Boundary Detection of Stents in Angiographic Images", IEEE Transact. on Medical Imaging, Vol. 19, No. 6, June 2000, Seiten 653-662, sowie in G. L. Turin: "An Introduction to Matched Filtering", IRE Trans. on Inform. Theory (June 1960), Seiten 311-329.

Gerade bei medizinischen Anwendungen sind indes die Dosisleistungen, mit welchen die Röntgenaufnahmen gemacht werden, stark zu begrenzen, um die Strahlenbelastung für den Patienten zu minimieren. Die hierdurch erzeugten Röntgenaufnahmen haben daher ein hohes Rauschen beziehungsweise eine entsprechend geringe Bildqualität, so dass sich die genannten besonders leistungsstarken Algorithmen hierauf nicht ohne weiteres anwenden lassen.

Als Ausweg aus dieser Situation wird bei dem erfindungsgemäßen Verfahren vorgeschlagen, im Bedarfsfalle eine Einzelaufnahme höherer Dosisleistung anzufertigen, welche eine hinreichende Bildqualität besitzt, um als Ausgangspunkt für Auswertungsalgorithmen zu dienen.

In der Figur ist als Beispiel die Situation veranschaulicht, dass die Bewegung eines Katheters beziehungsweise eines Führungsdrahtes im Körper eines Patienten verfolgt werden soll. Dabei kann in Schritt 1 des Verfahrens der untersuchende Arzt anzeigen, dass die Aufnahmen LD1 niedriger Dosis nicht mehr genügen und er eine Bildverbesserung beziehungsweise die Erkennung eines neuen Details des Objektes wünscht. Daraufhin wird in Schritt 2 ein Einzelbild HD mit erhöhter Dosisleistung erzeugt, welches dementsprechend ein geringes Rauschen bzw. eine höhere Bildqualität aufweist. Auf diesem Bild sind die gesuchten Details in der Regel gut zu erkennen.

Gemäß einer ersten Alternative kann in Schritt 3 der Arzt auf dem angefertigten Einzelbild HD anzeigen, welche Strukturen ihn genau interessieren. Diese Information wind dann in Schritt 5 dazu verwendet, in späteren, mit einer geringeren Dosis aufgenommenen Röntgenbildern LD2 dieses Detail wiederzuerkennen. Hierbei kommen insbesondere die oben genannten Matched-Filter Techniken zum Einsatz.

Alternativ zu Schritt 3 oder ergänzend hierzu kann in Schritt 4 auch an dem Einzelbild HD erhöhter Qualität eine automatische Mustererkennung durchgeführt werden. Da das Einzelbild eine hohe Qualität hat, können hier Algorithmen zum Einsatz kommen, welche gegenüber einem Rauschen nicht robust sind und daher für übliche fluoroskopische Aufnahmen LD1, LD2 nicht geeignet sind.

Die Erkenntnisse aus dem Einzelbild HD erhöhter Qualität können somit in Schritt 5 des Verfahrens bei der Detailverstärkung basierend auf der Kenntnis des Details vorteilhaft ausgenutzt werden.

Weiterhin kann in nachfolgenden Bildern LD2 eine Verarbeitung begrenzt auf einen interessierenden Bereich (ROI: Region of Interest) erfolgen, um die Darstellung in diesem Bereich, in dem sich das interessierende Objekt wie zum Beispiel ein Führungsdraht gerade befindet, zu verbessern. So kann zum Beispiel für diesen Bereich das lokale Histogramm der Grauwerte gestreckt werden.

## Patentansprüche

1. Verfahren zur Bildverarbeitung von Röntgenaufnahmen, wobei mindestens eine Röntgenaufnahme (HD) mit einer im Vergleich zu den übrigen Aufnahmen (LD1, LD2) höheren Dosisleistung hergestellt und bei der Bildverarbeitung der übrigen Aufnahmen (LD1, LD2) verwendet wird.

2. Verfahren nach Anspruch 1,
**dadurch gekennzeichnet,**
**dass** die Bildverarbeitung mit Hilfe eines Pattern-Matching-Algorithmus erfolgt, welcher mindestens ein Muster von einem darzustellenden Bilddetail verwendet.

3. Verfahren nach Anspruch 2,
**dadurch gekennzeichnet,**
**dass** der Pattern-Matching-Algorithmus eine automatische Mustererkennungsprozedur (4) verwendet, um das für die Verarbeitung von Aufnahmen (LD1, LD2) geringerer Dosisleistung benötigte Muster aus der Röntgenaufnahme (HD) höherer Dosisleistung zu gewinnen.

4. Verfahren nach einem der Ansprüche 2 oder 3,
**dadurch gekennzeichnet,**
**dass** der Pattern-Matching-Algorithmus eine Eingabeschnittstelle enthält, über welche das für die Verarbeitung von Aufnahmen (LD1, LD2) geringerer Dosisleistung benötigte Muster nach dessen Extraktion (3) aus der Röntgenaufnahme (HD) höherer Dosisleistung vorgegeben werden kann.

5. Verfahren nach mindestens einem der Ansprüche 1 bis 4,
**dadurch gekennzeichnet,**
**dass** die Bildverarbeitung von Röntgenaufnahmen (LD1, LD2) geringerer Dosisleistung sich auf die Bereiche (ROI) der Aufnahme konzentriert, in denen in der Röntgenaufnahme (HD) höherer Dosisleistung ein darzustellendes Detail detektiert wurde.

6. Verfahren nach mindestens einem der Ansprüche 1 bis 5,
**dadurch gekennzeichnet,**
**dass** eine Röntgenaufnahme (HD) höherer Dosisleistung aufgenommen wird, wenn ein Performance-Kriterium des zur Bildverarbeitung verwendeten Algorithmus eine vorgegebene Schwelle unterschreitet.

7. Vorrichtung zur Bildverarbeitung von digitalisierten Röntgenaufnahmen, enthaltend einen Zentralprozessor, der so eingerichtet ist, dass er ein Verfahren nach mindestens einem der Ansprüche 1 bis 6 ausführen kann.

8. Vorrichtung nach Anspruch 7,
**dadurch gekennzeichnet,**
**dass** der Zentral prozessor mit der Steuerung eines Röntgenaufnahmegerätes derart verbunden ist, dass er dieser eine Anforderung einer Röntgenaufnahme (HD) höherer Dosisleistung übermitteln kann.
